# EUROPEAN PATENT SPECIFICATION

(11) **EP 1 200 394 B1**
(45) Date of publication and mention of the grant of the patent: **27.09.2006**
(21) Application number: 00953844.8
(22) Date of filing: 04.08.2000
(51) Int. Cl.: C07C 255/00, C07C 69/76, C07C 235/00, C07C 41/00, C07D 303/38, C07C 253/30, C07D 303/48

(54) **PROCESS FOR PREPARING ACIDS VIA ALPHA-CHLOROEPOXY ESTERS**
EIN VERFAHREN ZUR HERSTELLUNG VON SÄUREN VIA ALPHA-CHLOROEPOXY-ESTHER
ELABORATION D'ACIDES PAR L'INTERMEDIAIRE D'ESTERS ALPHA-CHLOROEPOXY

(30) Priority: 06.08.1999 US 147576 P
(43) Date of publication of application: 02.05.2002
(73) Proprietor: SMITHKLINE BEECHAM CORPORATION, Philadelphia, PA 19103 (US)
(72) Inventor: DIEDERICH, Ann, M., Thorndale, PA 19372 (US); NOVAK, Vance, J., Devon, PA 19333 (US)
(74) Representative: Rutter, Keith
(86) International application number: PCT/US2000/021394
(87) International publication number: WO 2001/010822

(56) References cited:
- WO-A-95/24381
- WO-A2-98/34584
- US-A- 5 449 687
- US-A- 5 552 438
- US-A- 5 602 173
- US-A- 5 605 923
- CASTRO B. ET AL.: "Anion oxalyle. I.- Formation et stéreochimie d'un ester glycidique alpha-chloré dans la réaction de Darzens du dichloroacétate d'isopropyle sur la (5alpha)-cholestanone-3" BULLETIN DE LA SOCIETE CHIMIQUE DE FRANCE., no. 11, 1974, pages 2559-2563, XP002288770 FRSOCIETE FRANCAISE DE CHIMIE. PARIS.

## Description

This invention relates to a method for preparing certain acids which are useful as phosphodiesterase 4 inhibitors.

### Background of the Invention

The process of this invention relates to making compounds which are useful in treating diseases modulated by the isoforms of the phosphodiesterase 4 enzyme, and which are known PDE 4 inhibitors useful, among other things, for treating pulmonary diseases such as chronic obstructive pulmonary disease (COPD) and asthma.

International patent application with publication number WO 95/24381 (Smithkline Beecham Corporation) discloses certain compounds useful as PDE IV inhibitors and preparations thereof.

The compounds which are prepared by the methods of this invention and the intermediates disclosed herein are disclosed and described in the likes of U.S. patent 5,554,238 issued 03 September, 1996.
Those compounds, particularly the 4-cyanocyclohexanoic acids, have marked effects on neutrophil activity, inhibiting neutrophil chemotaxis and degranulation *in vitro.* In animal models, those compounds reduce neutrophil extravasation from the circulation, pulmonary sequestration and the edematous responses to a number of inflammatory insults *in vivo.*
They have been found to be useful in treating COPD in humans, and possibly in other mammalian species which suffer from COPD.

### Summary of the Invention

This invention relates to a method for enriching the cis form of a compound of Formula (IA) 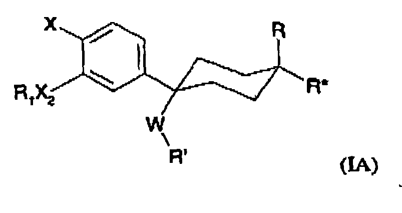 wherein:
R₁ is -(CR₄R₅)ₙC(O)O(CR₄R₅)ₘR₆, -(CR₄R₅)ₙC(O)NR₄(CR₄R₅)ₘR₆, -(CR₄R₅)ₙO(CR₄R₅)ₘR₆, or -(CR₄R₅)ᵣR₆ wherein the alkyl moieties are unsubstituted or substituted with one or more halogens;
m is 0 to 2;
n is 0 to 4;
r is 0 to 6;
R₄ and R₅ are independently selected hydrogen or C₁₋₂ alkyl;
R₆ is hydrogen, methyl, hydroxyl, aryl, halo substituted aryl, aryloxyC₁₋₃ alkyl, halo substituted aryloxyC₁₋₃ alkyl, indanyl, indenyl, C₇₋₁₁ polycycloalkyl, tetrahydrofuranyl, furanyl, tetrahydropyranyl, pyranyl, tetrahydrothienyl, thienyl, tetrahydrothiopyranyl, thiopyranyl, C₃₋₆ cycloalkyl, or a C₄₋₆ cycloalkyl containing one or two unsaturated bonds, wherein the cycloalkyl or heterocyclic moiety is unsubstituted or substituted by 1 to 3 methyl groups, one ethyl group, or an hydroxyl group;
   provided that:
   a) when R₆ is hydroxyl, then m is 2; or
   b) when R₆ is hydroxyl, then r is 2 to 6; or
   c) when R₆ is 2-tetrahydropyranyl, 2-tetrahydrothiopyranyl, 2-tetrahydrofuranyl, or 2-tetrahydrothienyl, then m is 1 or 2; or
   d) when R₆ is 2-tetrahydropyranyl, 2-tetrahydrothiopyranyl, 2-tetrahydrofuranyl,or 2-tetrahydrothienyl, then r is 1 to 6;
   e) when n is 1 and m is 0, then R₆ is other than H in -(CR₄R₅)ₙO(CR₄R₅)ₘR₆;
X is YR₂;
Y is O;
X₂ is O;
R₂ is -CH₃ or -CH₂CH₃, optionally substituted by I or more halogens;
R and R* are hydrogen or C(O)E wherein one of R or R* is always hydrogen and the other is always C(O)E where E is OR₁₄, or SR₁₄;
W is a bond or is alkenyl of 2 to 6 carbon atoms or alkynyl of 2 to 6 carbon atoms;
when W is a bond R' is hydrogen, halogen. C₁₋₄ alkyl, CH₂NHC(O)C(O)NH₂, halo-substituted C₁₋₄alkyl, CN, OR₈, CH₂OR₈, NR₈R₁₀, CH₂NR₈R₁₀, C(Z')H, C(O)OR₈, or C(O)NR₈R₁₀; and
when W is alkenyl of 2 to 6 carbon atoms or alkynyl of 2 to 6 carbon atoms then R' is COOR₁₄, C(O)NR₄R₁₄ or R₇;
R₇ is -(CR₄R₅)_{q}R₁₂ or C₁₋₆ alkyl wherein the R₁₂ or C₁₋₆ alkyl group is unsubstituted or substituted one or more times by: methyl or ethyl unsubstituted or substituted by 1-3 fluorines, or -F. -Br. -CI. -NO₂, -NR₁₀R₁₁, -C(O)R₈, -CO₂R₈, -O(CH₂)₂₋₄OR₈, -O(CH₂)_{q}R₈, -CN,-C(O)NR₁₀R₁₁, -O(CH₂)_{q}C(O)NR₁₀R₁₁,-O(CH₂)_{q}C(O)R₉, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)ₘR₉, -NR₁₀C(O)C(O)NR₁₀R₁₁, -NR₁₀C(O)C(O)R₁₀, or R₁₃;
q is 0. 1, or 2;
R₁₂ is R₁₃, C₃-C₇ cycloalkyl, or an unsubstituted or substituted aryl or heteroaryl group selected from the group consisting of (2-, 3- or 4-pyridyl), pyrimidyl, pyrazolyl, (1- or 2-imidazolyl), pyrrolyl, piperazinyl, piperidinyl, morpholinyl, furanyl, (2- or 3-thienyl), quinolinyl, naphthyl, and phenyl;
R₈ is independently selected from hydrogen or R₉;
R₉ is C₁₋₄ alkyl optionally substituted by one to three fluorines;
R₁₀ is OR₈ or R₁₁;
R₁₁ is hydrogen, or C₁₋₄ alkyl unsubstituted or substituted by one to three fluorines; or when R₁₀ and R₁₁ are as NR₁₀R₁₁ they may together with the nitrogen form a 5 to 7 membered ring comprised of carbon or carbon and one or more additional heteroatoms selected from O, N, or S;
R₁₃ is a substituted or unsubstituted heteroaryl group selected from the group consisting of oxazolidinyl, oxazolyl, thiazolyl, pyrazolyl, triazolyl, tetrazolyl, imidazolyl, imidazolidinyl, thiazolidinyl, isoxazolyl, oxadiazolyl, and thiadiazolyl, and where R₁₃ is substituted on R₁₂ or R₁₃ the rings are connected through a carbon atom and each second R₁₃ ring may be unsubstituted or substituted by one or two C₁₋₂ alkyl groups unsubstituted or substituted on the methyl with 1 to 3 fluoro atoms; and
R₁₄ is hydrogen;
which method comprises esterifying the acid or thioacid or converting them to a mixed anhydride, if they are not already in that form, then treating the ester, etc, with an alkoxide base for a time sufficient to give a ratio of *cis* to *trans* isomers which is at least 4:1, preferably 7:1 or greater.

### Detailed Description of the Invention

This invention provides a means for enriching the cis form of a cyclohexanoic acid in a mixture of *cis* and *trans* isomers.

As regards the preferred substituents on Formulas (IA), for R₁ they are CH₂-cyclopropyl or C₄₋₆ cycloalkyl. Preferred R₂ groups are a C₁₋₂ alkyl unsubstituted or substituted by 1 or more halogens. The halogen atoms are preferably fluorine and chlorine, more preferably fluorine. More preferred R₂ groups are those wherein R₂ is methyl, or a fluoro-substituted alkyl group, specifically a C₁₋₂ alkyl such as a -CF₃, -CHF₂, or -CH₂CHF₂. Most preferred are the -CHF₂ and -CH₃ moieties. Most preferred are those compounds wherein R₁ is -CH₂-cyclopropyl, cyclopentyl. 3-hydroxycyclopentyl, methyl or CHF₂ and R₂ is CF₂H or CH₃. Preferably the R₁₄ group will be methyl, ethyl or hydrogen. In formula (IA), methyl is the most preferred R₁₄ group, and in Formula (I), it is methyl or hydrogen. Particularly preferred are those compounds where R₁ is cyclopentyl and R₂ is CH₃.

As regards W, the preferred embodiment is where W is a bond, ethylenyl, or -C≡C-. When W is a bond, the preferred R' group is CN. And when W is ethylenyl, -C≡C- the preferred R' group is hydrogen.

The most preferred compound of Formula (IA) made by the process of this invention is *cis*-[4-cyano-4-(3-cyclopentyloxy-4-methoxyphenyl)cyclohexane-1-carboxylic acid].

Scheme I illustrates the conversion of a ketone of Formula (1-1) to the ester or acid of Formula (IA).

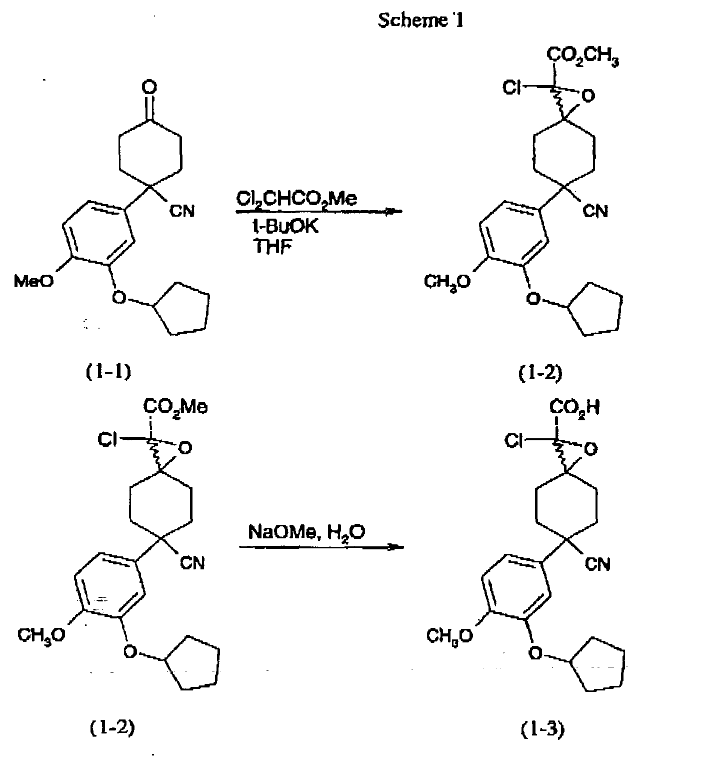

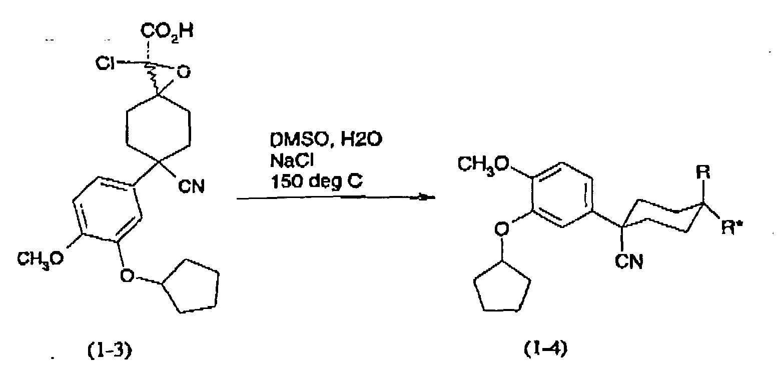

In compound 1-4, R and R* are hydrogen or C(O)OH, but R and R* are not both hydrogen or C(O)OH simultaneously.

The ketone starting material (1-1) can be prepared by the methods set out in U.S. patent 5,554,238 or 5,449,686. Forming the epoxide (1-2) is accomplished by treating the ketone with 1.1 to 2 equivalents of a lower alkyldihaloacetate using a polar non-protic solvent "Lower alkyl" here means an alkyl radical having 1-6 carbon atoms. It is preferred to use about 1.5 equivalents of the acetate, and teterahydrofuran as the solvent. First the ketone (1-1) and the actetate are dissolved in the solvent This solution is cooled to between -10 and +10 °C and an organic base is added in a molar excess (e.g. 1.1 to 2 equivalents, preferably about 1.5 equivalents). Herein an alkali metal *t*-butoxide is the preferred base, particularly potassium tert-butoxide. The temperature is kept at within the -10 to +10 °C range during the addition of the base and for some short period, 10 minutes to 45 minute thereafter. Product (1-2) is recovered by conventional means.

The ester (1-2) is then saponified using a base. This can be accomplished by any number of bases using conventional techniques. Herein this reaction is effected by treating the α-chloroepoxyester with sodium methoxide using a low molecular-weight alcohol and water as the solvent. A substantial molar excess of the base and solvent is used. For example a 5-fold excess of the base can be used and about a 10-fold excess of water. The ester is charged to a reaction vessel, dissolved in the alcohol, base is added and then the water is added The reaction goes to completion rapidly at room temperature, about 5 to 30 minutes. Product, the acid, is recovered by conventional means. Since it, the α-chlotoepoxy acid (1-3), is relatively unstable it is preferred to immediately treat the epoxide with a reactant which opens the ring to give the acid.

Herein the epoxy acid (1-3) is rearranged to give 1-4 using dimethyl sulfoxide and an alkali metal salt. Water is used as a co-sotvent. The alkali metal salt may be LiCl, KCl or NaCl, or the corresponding flouride and bromide salts LiF, KF, NaF, LiBr, KBr, and NaBr. By way of more specific example, the chloroepoxy acid is dissolved in dimethyl sulfoxide and water and a small amount of a sodium chloride is added to the reaction pot which is then heated for several hours. A preferred set of reactants and conditions is one where about a 10-fold excess of DMSO (by weight/volume) is used to dissolve the acid and a small amount of water and a salt such as sodium chloride is added. This solution is heated to between about 125 and 175 °C for 2-5 hours; preferably the solution is heated to about 150 °C for 3.5 hours or so. This reaction gives the cyclohexanoic acid as a mixture of the *cis* and *trans* isomers in about a 1-1 ratio.

Enrichment of the *cis* isomer in the mixture of *cis* and *trans* isomers obtained from the just-described reaction is accomplished by activating it by, for example, forming an ester or mixed anhydride, and then treating the ester with an alkoxide base, This technique can be applied with satisfactory results to any preparation where one has a mixture of isomers and wishes to enrich the *cis* form of the isomer in that mixture. By way of example, the technique used here is to esterify the acid using an acid and a lower alkanol to form the ester of the alkanol. Methanol is most perferred. This mixture is then treated with *t*-butanol and its alkali metal salt for an extended period, for between 5 and 24 hours for example; a preferred time is about 12 hours. This latter step results in an enrichment of the *cis* form of the product; the equilibration process gives the preferred *cis* form of the acid.

An alternative process is to combine the step of opening of the epoxy acid, really a decarboxylation, with the esterification step by using a lower alkanol or lower thioalkanol (1-6 carbons) as the co-solvent instead of water. The re-quilibration can be effected by adding the appropriate alcohol and its alkali metal salt to the reaction flask once the ester has been formed from the α-hatoepoxy acid without isolating the ester. For example methanol rather than water can be used as the solvent for the dimethyl sulfoxide/salt reaction. If this is done, one obtains the methyl ester as the product, rather than the acid obtained when water is used as the solvent. However if methanol or another low boiling-point alkanol is used, a pressurizable reaction vessel must be employed since the solution must be heated to about 150 °C to effect the decarboxylation, at which temperature the methanol would be mostly vaporized if the reaction was run at 1 atmosphere of pressure. A preferred approach is to run the reaction using methanol in a pressurized container, cooling the reaction mixture to about room temperature, and adding the likes of *t*-butanol and its alkali metal salt to effect the coversion of the *trans* form to the *cis* isomer.

By way of further illustration, but without intending to be limited in any way, the following illustrative examples are provided.

### Reference Examples and Examples

### Reference Example 1

### Preparation of methyl 2-chloro-6-cyano-6-[3-(cyclopentyloxy)-4-methoxyphenyl]-1-oxaspiro[2.5]octane-2-carboxylate

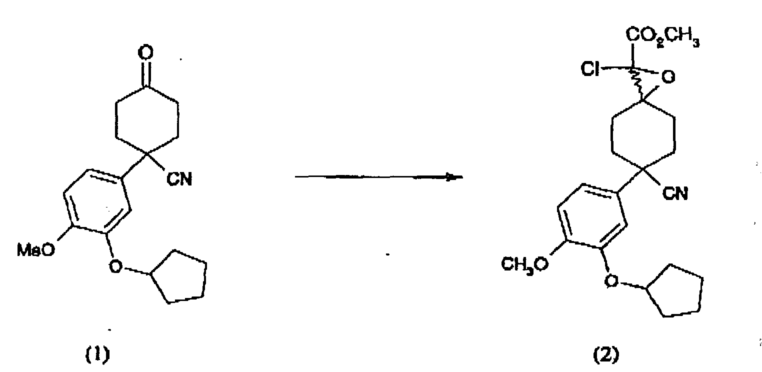

A 100 mL round-bottom flask was charged with 4-cyano-4-(3-cyclopentyloxy-4-methoxyphenyl)cyclohexan-1-one (1)(4.0 g, 12.8 mmole, 1.0 eq), methyldichloroacetate (2.74 g, 1.98 mL, 19.1 mmole, 1.5 eq), and tetrahydrofuran (THF, 40 mL). The solution was cooled to 0 °C in an ice bath, then potassium tert-butoxide was added (19.1 mL, 19.1 mmole of a 1M solution in THF) while maintaining the temperature below 5 °C (about 25 minutes). The reaction was deemed complete at the end by TLC (hexanes/ethyl acetate @3/1, silica gel plates), then was poured into ethyl acetate and 5% HCl for an extractive workup. The layers were separated and the water layer was extracted with ethyl acetate twice. The combined ethyl acetate layers were extracted with 5% sodium bicarbonate and with brine. The ethyl acetate layer was concentrated under vacuum to a yellow oil. The oil was dissolved in 3/1 hexanes/ethyl acetate and filtered through 1.5" of flash silica gel. Concentration produced the product methyl 2-chloro-6-cyano-6-[3-(cyclopentyloxy)-4-methoxyphenyl]-1-oxaspiro[2.5]octane-2-carboxylate as a clear, colorless oil. The molecular weight and structure of the product was confirmed to be the methyl α-chloroepoxy ester by mass spec.

### Reference Example 2

### Preparation of 2-chloro-6-cyano-6-[3-(cyclopentyloxy)-4-methoxyphenyl]-1-oxaspiro[2.5]octane-2-carboxylic acid

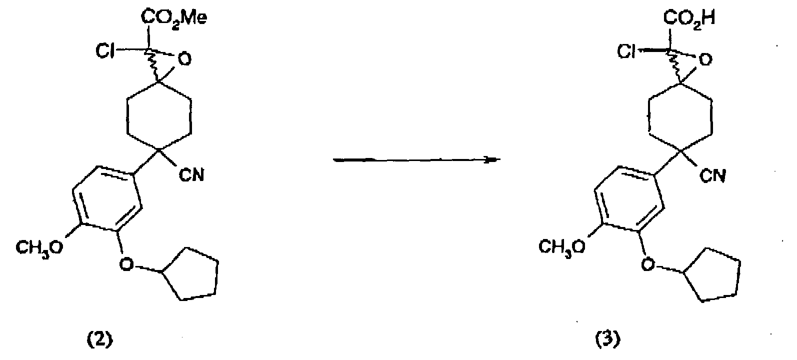

A 50 mL flask was charged with the chloroepoxyester (2) (3.0 g, 4.77 mmole), 30 mL of methanol, sodium methoxide (5.16 g of 25 wt % solution in methanol, 23.9 mmole) and water (0.8 g, 44 mmole). The solution was stirred for 10 minutes and the reaction was deemed complete by TLC (hexanestethyl acetate @3/1, silica gel plates). The reaction was poured into an addition funnel containing 100 mL of 1% HCl and 100 mL of *t*-butylmethyl ether. The organic layer was extracted once with water and once with brine, then was concentrated to an oil under reduced pressure. The product 2-chloro-6-cyano-6-[3-(cyclopentyloxy)-4-methoxyphenyl]-1-oxaspiro[2.5]octane-2-carboxylie acid was confirmed by mass spectral analysis.

### Reference Example 3

### Preparation of cis-[4-cyano-4-(3-cyclopentyloxy-4-methoxyphenyl)cyclohexane-1-carboxylic acid]

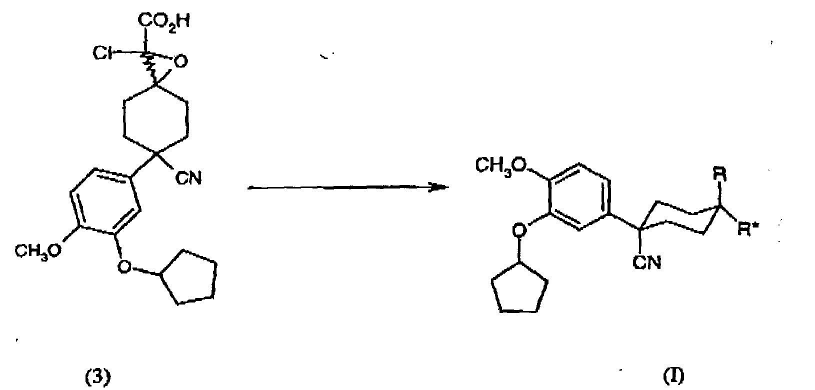

In this example, either R or R* can be C(O)OH; the other group must be hydrogen.

Freshly prepared chloroepoxyacid (3) (2.79 mmole) was treated with dimethyl sulfoxide (7.5 mL), water (0.5 mL), and NaCl (50 mg). The solution was heated to 150°C for 3.5 hours. The reaction was followed by HPLC (15 cm Supelcocil, ACN/water/TFA [40/60/0.1] 1.5 mL/min,215 nm UV, *trans* form - at 10.6 min and *cis* form at 11.3 min). The yield was calculated using weighted assay. The yield was 59% for the two isomers in a ratio of one-to-one.

### Example 4

### Enrichment of the Cis Isomer in a Cis/Trans Mixture of [4-cyano-4-(3-cyclopentyloxy-4-methoxyphenyl)cyclohexane-1-carboxylic acid]

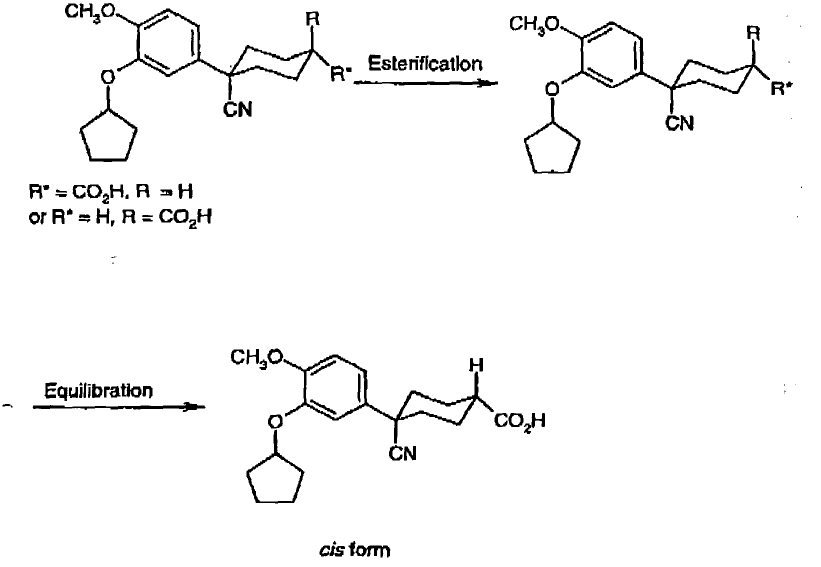

The isomeric mixture obtained in the previous step was dissolved in 10 mL of methanol. *p*-Toluenesulfonic acid (0,1 g) was added and the reaction was refluxed for 12 hours to form the methyl esters. The reaction was diluted with ethyl acetate and water. The layers were separated, then the organic layer was concentrated. The oil was dissolved in about 10 mL of tBuOH and then 7.5 mL of potassium *t*-butoxide (1M in t-BuOH) was added for the equilibration. After stirring overnight, a small sample was treated with water and the ratio of *cis* to trans isomers of [4-cyano-4-(3-cyclopentyloxy-4-methoxyphenyl)cyclohexane-1-carboxylic acid] was calculated as 9.6/1 by HPLC (15 cm Supelcocil, ACN/water/TFA [40/60/0.1] 1.5 mL/min,215 nm UV. *trans* form at 10.6 min and *cis* form at 11.3 min). The reaction was quenched by adding 1% HCl and ethyl acetate to extract. The layers were separated and the organic layer was extracted once with water. The product layer was concentrated and then treated with ethyl acetate. The product was precipitated by adding about one volume of hexanes. No *trans* form was detected in the product.

This reaction was also run using NaH under the same conditions. It gave an 8:1 ratio of *cis:trans* isomers. When the same reaction was run using NaH in ethanol (methyl ester) a 7:1 ratio was obtained. Using the ethyl ester rather than the methyl ester as the substrate, and NaH and ethanol, a 10:1 ratio was obtained.

### Reference Example 5

### One-Pot Preparation of the Cis-[4-cyano-4-(3-cyclopentyloxy-4-methoxyphenyl)cyclohexane-1-carboxylic acid] from Methyl 2-chloro-6-cyano-6-[3-(cyclopentyloxy)-4-methoxyphenyl]-1-oxaspiro[2.5]octane-2-carboxylate

Chloroepoxyester (0.72 g purifed, 1.71 mmole) in methanol (5 mL) was treated with sodium methoxide (1.42 g of 25% wt solution in methanol, 6.5 mmole) and water (0.5 mL) and stirred for 15 minutes. The reaction was quenched with *t*-butylmethyl ether and 1% HCl. The bottom layer was removed, then the organic layer was washed three times with water. The organic layer was concentrated under reduced pressure, then the water was azeotroped by adding methanol and reconcentrating.

Dimethylsulfoxide (7 mL), sodium chloride (0.5 g) and methanol (5 mL) were added. The contents were then heated under pressure to 150 °C for 1.5 hours. The HPLC (15 cm Supelcocil, ACN/water/TFA [40/60/0.1] 1.5 mL/min,215 nm UV) showed the isomeric mixture of esters and acids at 10.5/1 (esters/acids). The reaction was cooled, then 10 mL of t-BuOH and 0.20 g of t-BuOK was added The solution was stirred overnight to give a 7/1 ratio of *cis*/*trans* isomers. The reaction was worked up with 1% HCl and t-butylmethyl ether. The layers were separated and the organic layer was concentrated to an oil. The oil was dissolved in a minimum amount of warm ethyl acetate, and the product was precipitated by adding hexanes, cooled to 0 °C, then filtered. The product was a light tan solid; no trans isomer was detected.

## Claims

1. A process for enriching the *cis* form of a compound of Formula (IA) 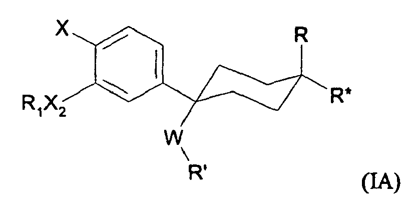 wherein:
R₁ is -(CR₄R₅)ₙC(O)O(CR₄R₅)ₘR₆, -(CR₄R₅)ₙC(O)NR₄(CR₄R₅)ₘR₆, -(CR₄R₅)ₙO(CR₄R₅)ₘR₆, or -(CR₄R₅)ᵣR₆ wherein the alkyl moieties unsubstituted or substituted with one or more halogens;
m is 0 to 2;
n is 0 to 4;
r is 0 to 6;
R₄ and R₅ are independently selected hydrogen or C₁₋₂ alkyl;
R₆ is hydrogen, methyl, hydroxyl, aryl, halo substituted aryl, aryloxyC₁₋₃ alkyl, halo substituted aryloxyC₁₋₃ alkyl, indanyl, indenyl, C₇₋₁₁ polycycloalkyl, tetrahydrofuranyl, furanyl, tetrahydropyranyl, pyranyl, tetrahydrothienyl, thienyl, tetrahydrothiopyranyl, thiopyranyl, C₃₋₆ cycloalkyl, or a C₄₋₆ cycloalkyl containing one or two unsaturated bonds, wherein the cycloalkyl or heterocyclic moiety is unsubstituted or substituted by 1 to 3 methyl groups, one ethyl group, or an hydroxyl group;
provided that:
a) when R₆ is hydroxyl, then m is 2; or
b) when R₆ is hydroxyl, then r is 2 to 6; or
c) when R₆ is 2-tetrahydropyranyl, 2-tetrahydrothiopyranyl, 2-tetrahydrofuranyl, or 2-tetrahydrothienyl, then m is 1 or 2; or
d) when R₆ is 2-tetrahydropyranyl, 2-tetrahydrothiopyranyl, 2-tetrahydrofuranyl,or 2-tetrahydrothienyl, then r is 1 to 6;
e) when n is 1 and m is 0, then R₆ is other than H in -(CR₄R₅)ₙO(CR₄R₅)ₘR₆;
X is YR₂;
Y is O;
X₂ is O;
R₂ is -CH₃ or -CH₂CH₃, optionally substituted by 1 or more halogens;
R and R* are hydrogen or C(O)E wherein one of R or R* is always hydrogen and the other is always C(O)E where E is OR₁₄ or SR₁₄ and R₁₄ is hydrogen;
W is a bond or is alkenyl of 2 to 6 carbon atoms or alkynyl of 2 to 6 carbon atoms;
when W is a bond R' is hydrogen, halogen, C₁₋₄ alkyl, CH₂NHC(O)C(O)NH₂, halo-substituted C₁₋₄ alkyl, CN, OR₈, CH₂OR₈, NR₈R₁₀, CH₂NR₈R₁₀, C(Z')H, C(O)OR₈, or C(O)NR₈R₁₀; and
when W is alkenyl of 2 to 6 carbon atoms or alkynyl of 2 to 6 carbon atoms then R' is R' is COOR₁₄, C(O)NR₄R₁₄ or R₇;
R₇ is -(CR₄R₅)_{q}R₁₂ or C₁₋₆ alkyl wherein the R₁₂ or C₁₋₆ alkyl group is unsubstituted or substituted one or more times by: methyl or ethyl unsubstituted or substituted by 1-3 fluorines, -F, -Br, -Cl, -NO₂, -NR₁₀R₁₁, -C(O)R₈, -CO₂R₈, -O(CH₂)₂₋₄OR₈, -O(CH₂)_{q}R₈, -CN, -C(O)NR₁₀R₁₁, -O(CH₂)_{q}C(O)NR₁₀R₁₁, - O(CH₂)_{q}C(O)R₉, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)_{m'}R₉, -NR₁₀C(O)C(O)NR₁₀R₁₁, -NR₁₀C(O)C(O)R₁₀, or R₁₃;
q is 0, 1, or 2;
R₁₂ is R₁₃, C₃-C₇ cycloalkyl, or an unsubstituted or substituted aryl or heteroaryl group selected from the group consisting of (2-, 3- or 4-pyridyl), pyrimidyl, pyrazolyl, (1- or 2-imidazolyl), pyrrolyl, piperazinyl, piperidinyl, morpholinyl, furanyl, (2- or 3-thienyl), quinolinyl, naphthyl, and phenyl;
R₈ is independently selected from hydrogen or R₉;
R₉ is C₁₋₄ alkyl optionally substituted by one to three fluorines;
R₁₀ is OR₈ or R₁₁;
R₁₁ is hydrogen, or C₁₋₄ alkyl unsubstituted or substituted by one to three fluorines; or when R₁₀ and R₁₁ are as NR₁₀R₁₁ they may together with the nitrogen form a 5 to 7 membered ring comprised of carbon or carbon and one or more additional heteroatoms selected from O, N, or S;
R₁₃ is a substituted or unsubstituted heteroaryl group selected from the group consisting of oxazolidinyl, oxazolyl, thiazolyl, pyrazolyl, triazolyl, tetrazolyl, imidazolyl, imidazolidinyl, thiazolidinyl, isoxazolyl, oxadiazolyl, and thiadiazolyl, and where R₁₃ is substituted on R₁₂ or R₁₃ the rings are connected through a carbon atom and each second R₁₃ ring may be unsubstituted or substituted by one or two C₁₋₂ alkyl groups unsubstituted or substituted on the methyl with 1 to 3 fluoro atoms;
which process comprises formation of the C₁₋₆ alkyl ester, C₁₋₆ alkyl thioester or mixed anhydride of Formula (IA), followed by treatment with an alkoxide base.

2. The process of claim 1 wherein the compound of formula IA, R₁ is CH₂₋cyclopropyl, CH₂-C₅₋₆ cycloalkyl, or C₄₋₆ cycloalkyl, R₂ is C₁₋₂ alkyl unsubstituted or substituted by 1 or more halogens, the base is a alkali metal t-butoxide, and the reaction runs for 5-24 hours.

3. The process of claim 1 wherein the compound of formula IA is [4-cyano-4-(3-cyclopentyloxy-4-methoxyphenyl)cyclohexane-1-carboxylic acid].

4. The process of claim 1 wherein the base is potassium *t*-butoxide.

## Patentansprüche

1. Verfahren zum Anreichern der cis-Form einer Verbindung der Formel (IA) 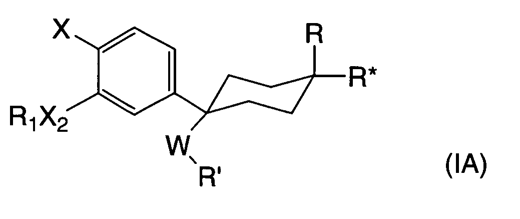 wobei:
R₁ -(CR₄R₅)ₙC(O)O(CR₄R₅)ₘR₆, -(CR₄R₅)ₙC(O)NR₄(CR₄R₅)ₘR₆, -(CR₄R₅)ₙO(CR₄R₅)ₘR₆ oder -(CR₄R₅)ᵣR₆, wobei die Alkylreste nicht substituiert sind oder mit einem oder mehreren Halogenen substituiert sind;
m ist 0 bis 2;
n ist 0 bis 4;
r ist 0 bis 6;
R₄ und R₅ werden unabhängig voneinander ausgewählt aus Wasserstoff oder C₁₋₂-Alkyl;
R₆ ist Wasserstoff, Methyl, Hydroxyl, Aryl, Halo-substituiertes Aryl, Aryloxy-C₁₋₃-Alkyl, Halo-substituiertes Aryloxy-C₁₋₃-Alkyl, Indanyl, Indenyl, C₇₋₁₁-Polycycloalkyl, Tetrahydrofuranyl, Furanyl, Tetrahydropyranyl, Pyranyl, Tetrahydrothienyl, Thienyl, Tetrahydrothiopyranyl, Thiopyranyl, C₃₋₆-Cyclocalkyl oder ein C₄₋₆-Cycloalkyl, das eine oder zwei ungesättigte Bindungen enthält, wobei der Cycloalkyl- oder heterozyklische Rest nicht substituiert ist oder durch eine bis drei Methylgruppen, eine Ethylgruppe oder eine Hydroxylgruppe substituiert ist;
mit der Maßgabe, dass:
a) wenn R₆ Hydroxyl ist, dann ist m 2; oder
b) wenn R₆ Hydroxyl ist, dann ist r 2 bis 6; oder
c) wenn R₆ 2-Tetrahydropyranyl, 2-Tetrahydrothiopyranyl, 2-Tetrahydrofuranyl oder 2-Tetrahydrothienyl ist, dann ist m 1 oder 2; oder
d) wenn R₆ 2-Tetrahydropranyl, 2-Tetrahydrothiopyranyl, 2-Tetrahydrofuranyl oder 2-Tetrahydrothienyl ist, dann ist r 1 bis 6;
e) wenn n 1 ist und m 0 ist, dann ist R₆ verschieden von H in -(CR₄R₅)ₙO(CR₄R₅)ₘR₆;
X ist YR₂;
Y ist O;
X₂ ist O;
R₂ ist -CH₃ oder -CH₂CH₃, gegebenenfalls substituiert durch ein oder mehrere Halogene;
R und R* sind Wasserstoff oder C(O)E, wobei eines von R oder R* immer Wasserstoff ist und das andere immer C(O)E ist, worin E OR₁₄ ist oder SR₁₄ ist;
W ist eine Bindung oder ist ein Alkenyl mit 2 bis 6 Kohlenstoffatomen oder Alkynyl mit 2 bis 6 Kohlenstoffatomen; wenn W eine Bindung ist, ist R' Wasserstoff, Halogen, C₁₋₄-Alkyl, CH₂NHC(O)C(O)NH₂; Halo-substituiertes C₁₋₄-Alkyl, CN, OR₈, CH₂OR₈, NR₈R₁₀, CH₂NR₈R₁₀, C(Z)H, C(O)OR₈ oder C(O)NR₈R₁₀; und
wenn W Alkenyl mit 2 bis 6 Kohlenstoffatomen oder Alkynyl mit 2 bis 6 Kohlenstoffatomen ist, dann ist R' COOR₁₄, C(O)NR₄R₁₄ oder R₇;
R₇ ist -(CR₄R₅)_{q}R₁₂ oder C₁₋₆-Alkyl, wobei R₁₂ oder die C₁₋₆-Alkylgruppe nicht substituiert ist oder einmal oder mehrere Male substituiert ist durch: Methyl oder Ethyl, die nicht substituiert sind oder mit 1-3-Fluor substituiert sind, oder mit -F, -Br, -Cl, -NO₂, -NR₁₀R₁₁, -C(O)R₈, -CO₂R₈, -O(CH₂)₂₋₄OR₈, -O(CH₂)_{q}R₈, -CN, -C(O)NR₁₀R₁₁, -O(CH₂)_{q}C(O)NR₁₀R₁₁, -O(CH₂)_{q}C(O)R₉, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)ₘR₉, -NR₁₀C(O)C(O)NR₁₀R₁₁, -NR₁₀C(O)C(O)R₁₀ oder R₁₃;
q ist 0, 1 oder 2;
R₁₂ ist R₁₃, C₃₋₇-Cycloalkyl oder eine nicht substituierte oder substituierte Aryl- oder Heteroarylgruppe, ausgewählt aus der Gruppe bestehend aus (2-, 3- oder 4-Pyridyl), Pyrimidyl, Pyrazolyl, (1- oder 2-Imidazolyl), Pyrrolyl, Piperazinyl, Piperidinyl, Morpholinyl, Furanyl, (2- oder 3-Thienyl), Chinolinyl, Naphthyl und Phenyl;
R₈ wird unabhängig ausgewählt aus Wasserstoff oder R₉;
R₉ ist C₁₋₄-Alkyl, gegebenenfalls substituiert mit 1 bis 3 Fluoratomen;
R₁₀ ist OR₈ oder R₁₁;
R₁₁ ist Wasserstoff oder C₁₋₄-Alkyl, das nicht substituiert ist oder mit 1 bis 3 Fluoratomen substituiert ist; oder wenn R₁₀ und R₁₁ wie NR₁₀R₁₁ sind, können sie zusammen mit dem Stickstoff einen 5- bis 7-gliedrigen Ring bilden, der aus Kohlenstoff zusammengesetzt ist oder aus Kohlenstoff und einem oder mehreren zusätzlichen Heteroatomen, ausgewählt aus O, N oder S;
R₁₃ ist eine substituierte oder nicht substituierte Heteroarylgruppe, ausgewählt aus der Gruppe bestehend aus Oxazolidinyl, Oxazolyl, Thiazolyl, Pyrazolyl, Triazolyl, Tetrazolyl, Imidazolyl, Imidazolidinyl, Thiazolidinyl, Isoxazolyl, Oxadiazolyl und Thiadiazolyl, und wenn R₁₃ an R₁₂ oder R₁₃ substituiert ist, sind die Ringe durch ein Kohlenstoffatom verbunden und jeder zweite R₁₃-Ring kann nicht substituiert sein oder durch 1 oder 2 C₁₋₂-Alkylgruppen substituiert sein, die nicht substituiert sind oder am Methyl mit 1 bis 3 Fluoratomen substituiert sind; und
R₁₄ ist Wasserstoff;
wobei das Verfahren die Bildung des C₁₋₆-Alkylesters, des C₁₋₆-Alkylthioesters oder eines gemischten Anhydrids der Formel (IA) gefolgt von der Behandlung mit einer Alkoxidbase umfasst.

2. Verfahren gemäß Anspruch 1, wobei die Verbindung der Formel IA, R₁ CH₂-Cyclopropyl, CH₂-C₅₋₆-Cycloalkyl oder C₄₋₆-Cycloalkyl, R₂ C₁₋₂-Alkyl ist, das nicht substituiert ist oder mit einem oder mehreren Halogenen substituiert ist, die Base ein Alkalimetall-t-butoxid ist, und die Reaktion für 5 bis 24 Stunden abläuft.

3. Verfahren gemäß Anspruch 1, wobei die Verbindung der Formel IA [4-Cyano-4-(3-cyclopentyloxy-4-methoxyphenyl)cyclohexan-1-carbonsäure] ist.

4. Verfahren gemäß Anspruch 1, wobei die Base Kalium-t-butoxid ist.

## Revendications

1. Procédé d'enrichissement en forme-*cis* d'un composé de Formule (IA) : 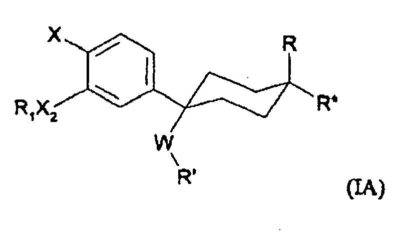 dans laquelle :
- R₁ représente un groupe -(CR₄R₅)ₙC(O)O(CR₄R₅)ₘR₆, -(CR₄R₅)ₙC(O)NR₄(CR₄R₅)ₘR₆, -(CR₄R₅)ₙO(CR₄R₅)ₘR₆, ou -(CR₄R₅)ᵣR₆ dans lequel les entités alkyle sont non substituées ou substituées par un ou plusieurs halogènes;
- m est de 0 à 2 ;
- n est de 0 à 4 ;
- r est de 0 à 6 ;
- R₄ et R₅ sont choisis indépendamment entre l'hydrogène ou un groupe alkyle C₁₋₂;
- R₆ représente l'hydrogène, un groupe méthyle, hydroxyle, aryle, aryle substitué par un halogène, aryloxyalkyle C₁₋₃, aryloxyalkyle C₁₋₃ substitué par un halogène, indanyle, indényle, polycycloalkyle C₇₋₁₁, tétrahydrofuranyle, furanyle, tétrahydropyranyle, pyranyle, tétrahydrothiényle, thiényle, tétrahydrothiopyranyle, thiopyranyle, cycloalkyle C₃₋₆ ou un groupe cycloalkyle C₄₋₆ contenant une ou deux liaisons insaturées, où l'entité cycloalkyle ou hétérocyclique est non substituée ou substituée par 1 à 3 groupes méthyle, un groupe éthyle ou un groupe hydroxyle ;
à la condition que
(a) lorsque R₆ représente un hydroxyle, m soit alors égal à 2 ; ou
(b) lorsque R₆ représente un hydroxyle, r soit alors de 2 à 6 ; ou
(c) lorsque R⁶ représente le 2-tétrahydropyranyle, le 2-tétrahydrothiopyranyle, le 2-tétrahydrofuranyle ou le 2-tétrahydrothiényle, m soit alors égal à 1 ou à 2 ; ou
(d) lorsque R⁶ représente le 2-tétrahydropyranyle, le 2-tétrahydrothiopyranyle, le 2-tétrahydrofuranyle ou le 2-tétrahydrothiényle, r soit alors de 1 à 6 ;
(e) lorsque n est égal à 1 et m est égal 0, R₆ soit alors autre que H dans le groupe -(CR₄R₅)ₙO(CR₄R₅)ₘR₆ ;
- X représente YR₂ ;
- Y représente O ;
- X₂ représente O ;
- R₂ représente un groupe -CH₃ ou -CH₂CH₃ éventuellement substitué par un ou plusieurs halogènes ;
- R et R* représentent l'hydrogène ou un groupe C(O)E dans lequel l'un de R ou R* représente toujours l'hydrogène et l'autre représente toujours C(O)E dans lequel E est OR₁₄ ou SR₁₄ et R₁₄ représente l'hydrogène ;
- W est une liaison ou représente un groupe alkényle de 2 à 6 atomes de carbone ou un groupe alkynyle de 2 à 6 atomes de carbone ;
lorsque W est une liaison, R' représente l'hydrogène, un halogène, un groupe alkyle C₁₋₄, CH₂NHC(O)C(O)NH₂, alkyle C₁₋₄ substitué par un halogène, CN, OR₈, CH₂OR₈, NR₈R₁₀, CH₂NR₈R₁₀, C(Z')H, C(O)OR₈ ou C(O)NR₈R₁₀; et
lorsque W représente un groupe alkényle de 2 à 6 atomes de carbone ou un groupe alkynyle de 2 à 6 atomes de carbone, R' est alors R' et représente un groupe COOR₁₄, C(O)NR₄R₁₄ ou R₇;
- R₇ représente un groupe -(CR₄R₅)_{q}R₁₂ ou alkyle C₁₋₆ dans lequel le groupe R₁₂ ou alkyle C₁₋₆ est non substitué ou substitué une ou plusieurs fois par : un méthyle ou éthyle non substitué ou substitué par 1 à 3 atomes de fluor, -F, -Br, -Cl, -NO₂, -NR₁₀R₁₁, -C(O)R₈, -CO₂R₈, -O(CH₂)₂₋₄OR₈, -O(CH₂)_{q}R₈, -CN, -C(O)NR₁₀R₁₁, -O(CH₂)_{q}C(O)NR₁₀R₁₁, -O(CH₂)_{q}C(O)R₉, -NR₁₀C(O)NR₁₀R₁₁, -NR₁₀C(O)R₁₁, -NR₁₀C(O)OR₉, -NR₁₀C(O)R₁₃, -C(NR₁₀)NR₁₀R₁₁, -C(NCN)NR₁₀R₁₁, -C(NCN)SR₉, -NR₁₀C(NCN)SR₉, -NR₁₀C(NCN)NR₁₀R₁₁, -NR₁₀S(O)₂R₉, -S(O)ₘR₉, -NR₁₀C(O)C(O)NR₁₀R₁₁, -NR₁₀C(O)C(O)R₁₀ ou R₁₃;
- q est égal à 0, 1 ou 2 ;
- R₁₂ représente R₁₃, un groupe cycloalkyle C₃-C₇ ou un groupe aryle ou hétéroaryle non substitué ou substitué, choisi au sein du groupe consistant en des groupes 2-, 3- ou 4-pyridyle, pyrimidyle, pyrazolyle, 1-ou 2-imidazolyle, pyrrolyle, pipérazinyle, pipéridinyle, morpholinyle, furanyle, 2- ou 3-thiényle, quinoléinyle, naphtyle et phényle ;
- R₈ est choisi indépendamment parmi l'hydrogène ou R₉;
- R₉, représente un groupe alkyle C₁₋₄ éventuellement substitué par un à trois atomes de fluor ;
- R₁₀ représente OR₈ ou R₁₁;
- R₁₁ représente l'hydrogène ou un groupe alkyle C₁₋₄ non substitué ou substitué par un à trois atomes de fluor ; ou bien lorsque R₁₀ et R₁₁ sont sous la forme NR₁₀R₁₁ ils peuvent, en association avec l'azote, former un cycle à 5 à 7 atomes composé de carbone ou de carbone avec un ou plusieurs hétéroatomes additionnels choisis parmi O, N ou S ;
- R₁₃ représente un groupe hétéroaryle substitué ou non substitué choisi au sein du groupe consistant en des groupes oxazolidinyle, oxazolyle, thiazolyle, pyrazolyle, triazolyle, tétrazolyle, imidazolyle, imidazolidinyle, thiazolidinyle, isoxazolyle, oxadiazolyle et thiadiazolyle, et là où R₁₃ est substitué sur R₁₂ ou R₁₃, les cycles sont reliés par un atome de carbone et chaque second cycle de R₁₃ peut être non substitué ou substitué par un ou deux groupes alkyle C₁₋₂ non substitués ou substitués sur le méthyle par 1 à 3 atomes de fluor
lequel procédé comprend la formation de l'alkyl(C₁₋₆)ester, de l'alkyl-(C₁₋₆)thioester ou de l'anhydride mixte de Formule (IA), suivie d'un traitement avec une base alcoxyde.

2. Procédé selon la revendication 1, dans lequel, dans le composé de formule (IA), R₁ représente un groupe CH₂-cyclopropyle, CH₂-cycloalkyle C₅₋₆ ou cycloalkyle C₄₋₆, R₂ représente un groupe alkyle C₁₋₂ non substitué ou substitué par 1 ou plusieurs halogènes, la base est un ter-butoxyde de métal alcalin et la réaction dure de 5 à 24 heures.

3. Procédé selon la revendication 1, dans lequel le composé de formule (IA) est l'acide 4-cyano-4-(3-cyclopentyloxy-4-méthoxyphényl)cyclohexane-1-carboxylique.

4. Procédé selon la revendication 1, dans lequel la base est le tert-butoxyde de potassium.
